# EUROPEAN PATENT SPECIFICATION

(11) **EP 0 640 350 B1**
(45) Date of publication and mention of the grant of the patent: **10.04.2002**
(21) Application number: 94116972.4
(22) Date of filing: 16.09.1992
(51) Int. Cl.: A61K 47/48, A61K 49/00

(54) **Ceramic particles and their preparation**
Keramische Partikel und Verfahren zur ihren Herstellung
Particules de céramique et leur préparation

(30) Priority: 16.09.1991 GB 9119762
(43) Date of publication of application: 01.03.1995
(62) Divisional of application: 92919662.4
(73) Proprietor: SYNGENIX LIMITED, Cambridge CB3 0BL (GB)
(72) Inventor: Filler, Aaron Gershon, London, SW20 0NE (GB); Lever, Andrew Michael Lindsay, Cambridge, CB1 4SL (GB)
(74) Representative: Roques, Sarah Elizabeth

(56) References cited:
- EP-A- 0 040 512
- EP-A- 0 040 722
- WO-A-88/07365
- WO-A-89/03675
- WO-A-92/04916
- WO-A-92/05250
- WO-A-92/11037
- WO-A-92/11846
- GB-A- 2 221 466
- CHEMICAL ABSTRACTS, vol. 104, no. 7, 17 February 1986, Columbus, Ohio, US; abstract no. 48280, PAUL KRONICK ET AL. 'Use of superparamagnetic particles for isolation of cells' & J. BIOCHEM. BIOPHYS. METHODS, vol.12, 1986 pages 73 - 80 'CODEN: JBBMDG;ISSN: 0165-022x'

## Description

This invention relates to ceramic particles of the type that can be used for therapeutic or diagnostic purposes, and to their preparation.

The preparation of inorganic, metal oxide particles for therapeutic delivery is described in WO-A-9204916. Such particles can be prepared with a dextran or other polymer coating. The particles may be prepared by coprecipitation of ferrous and ferric salts from aqueous solution.

Kronick et al, J. Biochem. Biophys. Methods 12 (1986) 73-80, also discloses particles produced by precipitation from ferrous/ferric ions in the presence of dextran.

According to the present invention, known such particles, coated with a biologically - tolerable polymer such as dextran, are free of hydrous oxides. As illustrated in the Example, the preparation of the particles gives desirable homogeneity and avoidance of water-soluble materials that may adversely affect the desired slow metabolism. The Example obviates column chromatography, for which the use of centrifugal concentrators has been substituted, and involves the corresponding omission of NaCl elution buffers. EDTA is used as a chelating agent in the buffer used in the first washing steps. This apparently dissolves the iron in the hydrous oxides, but does not dissolve the well-formed ferrites. The result is a stable and uniform particle preparation with low toxicity (because it is a substantially pure ceramic preparation).

DNA, RNA, plasmids, ribosomal particles, nucleic acid binding-proteins, and any other necessary molecules may be caused to adhere to the outer surface of any one of a variety of metal oxide or mixed metal crystals of coated or uncoated type or to be attached to the surface of or included in the body of a variety of other types of biodegradable particles of appropriate size and capable of surface attachment to a cell adhesion molecule. There may be included on the surface one of a variety of nerve adhesion molecules or muscle adhesion molecules which bind to the surface of nerve and muscle cells, but preferably to muscle cells.

When such particles are constructed and then administered by routine percutaneous intramuscular injection, an exceedingly safe and efficient transfection process is initiated. The particles adhere to the outer surfaces of muscle cells and to the outer surfaces of the axon termini of motor nerve cells or preferably to the dendritic or sensory process of sensory axons within the muscle. After adherence, the particles are ingested into the nerve and muscle cells by a natural process termed adsorptive endocytosis.

The metal oxide core may be constructed in such a way as to demonstrate superparamagnetism; external magnetic fields (as from US-A-4869247) can then be used to aid in targeting the agents.

Nucleic acid attachment to the particle may be effected by specific nucleic acid-binding proteins. A DNA plasmid or strand is constructed to include both the desired treatment gene and a segment with very high affinity for a selected nucleic acid-binding protein. This pairing can be optimised by binding the attachment DNA segment to immobilise latex particles using a cyanogen bromide immobilisation technique. Various nucleic acid binding proteins and other cell constituents are then passed through an affinity column made up to such DNA tagged latex particles. The specific fraction of nucleic acid binding protein is then eluted for use in making the particle.

Dextran or other coating of the particle may be used as a framework to which various other types of molecules are then covalently bound. Typically, a targeting molecule such as an antibody or antibody fragment, or some other useful cell adhesion molecule is used. This causes the particle to adhere selectively to certain desirable cell types, e.g. a gp120 fragment to promote adherence to CD4 positive cells. In addition to the targeting molecule, it is also possible to attach a nucleic acid-binding protein or short cDNA sequence to the dextran coat. In this fashion, particles can be produced with appropriate nucleic acid binding proteins and targeting molecules, and then subsequently loaded with the therapeutic DNA.

To prepare the particles, a mixture of ferrous and ferric chloride salts may be dissolved in a saturated dextran solution after the fashion of US-A-4452773 and precipitated by addition of 7.5% ammonia solution. In another example of the preparation, the initial precipitation of the iron salts is done by dropwise addition to ammonia solution without the presence of any coating dextran or other molecule. The resulting suspension is spun in a centrifuge at 500 g for 10 minutes and the pellet washed and resuspended in distilled water and the process then repeated but with a wash with 0.01N HCl. The resulting stable colloid is then exposed to a mixture of adhesion molecule protein, nucleic acid strands and/or nucleic acid binding proteins. After an incubation with gentle non-magnetic stirring for one hour, the remaining reactive sites on the particles are blocked by the addition of dextran or albumin protein. The particles are then passed through Sephadex® 150 and Sephacryl® 200 columns then affinity-purified by means of the cell adhesion molecule using for instance a column of affinity-labelled agarose, Sepharose®, or latex beads.

In yet another example of the preparation, the initial precipitation is carried out by preparing a solution of very strong buffer such as 1 Molar or higher concentration of HEPES or Tris at a pH of 7.4. The nucleic acids, any desired dextran, and or targeting proteins and nucleic acid binding proteins are added directly to this initial strong buffer. The mixture of dissolved ferrous and ferric iron salts in aqueous solution or in a solution containing dextran and/or protein and/or nucleic acids is then added dropwise to the buffer solution. In this fashion, the particles are formed in a rigidly buffered solution and so many fragile protein and peptide molecules can be used to form the particle coat where such molecules are necessary for targeting, for introducing ribonucleoprotein or ribosomal protein or other aspects of transcription signalling or actual transcription mechanism proteins along with the DNA or RNA. The product of this precipitation reaction is then further blocked with dextran or albumin if necessary, then purified with Sephadex® 150, Sephacryl® 200, Amicon ultrafilters and affinity columns as described above.

In yet another version of the synthesis, there is no nucleic acid binding protein used but only a cell surface adhesion molecule. Instead of the nucleic acid binding protein, a complementary fragment of the nucleic acid of interest is bound to the particles by a cyanogen bromide or other type of binding reaction or by adherence to an uncoated particle type. A gene of interest is then attached to the particle by its interaction with the bound complementary fragment after which purification steps are carried out as described above.

In summary, the present invention provides a synthetic transfection agent, the corresponding vector without the nucleic acid, and any combination of the components thereof. It will be appreciated that the synthetic transfection agent is based on precipitation of ceramic metal oxide particles similar in size to a virus. The metal oxide particle may be coated with dextran or other biologically-tolerable polymer during the precipitation process. Chemically, the basic structure is similar to drugs in current use as magnetic resonance contrast agents.

For intravascular administration, the particle size determines serum half-life and destination. Larger particles tend to be cleared into the reticuloendothelial cells by phagocytosis, while small particles achieve destinations determined more completely by their targeting molecule.

These particles can also be administered intramuscularly where they can gain entry into muscle cells and also can be ingested by nerve terminals in the muscle and subsequently subjected to axonal transport from the periphery towards the neural cell bodies in the central nervous system. In this fashion, mimicking the route of the Herpes virus, an intramuscular injection can be used to deliver DNA across the blood-brain barrier for therapeutic purposes in selected regions of the nervous system. The axonal transport route also provides access to Schwann cells which line the axons.

It is further possible to provide the particles in aerosol form for pulmonary administration. A variety of other routes of administration are also feasible, including intravenous administration.

The particulate carrier is well suited for treating diseases involving the reticuloendothelial system through intravascular and inhalational routes, and to treat GI mucosal cells by enteral routes, as well as for intramuscular injection for access to muscle cells. Access via the intraneural route, to CNS and ganglion cells, is provided by intramuscular and intradermal injection.

The particles are biodegradable in the sense that they can break down, in vivo, to materials that are essentially harmless. Thus, for example, while foreign materials such as gold particles may be found intact in cells years later, iron oxide particles dissolve readily into oxygen and iron, both of which are of course naturally present in abundance in cells and which then participate in normal cellular metabolism, storage and reuse. Iron poses some risk of toxicity when present in high amounts. The potential toxicity of ferrites is reduced by ensuring that they dissolve slowly, at a rate no faster than the cells' ability to process the elemental iron. Extension of the degradation rate for the particles can be achieved by ensuring that the original preparation is free of hydrous oxides of iron that are similar in overall size and chemical composition to ferrites but dissolve very rapidly. Additionally, by doping in other elements such as palladium (which is water-soluble in elemental form unlike gold) which can improve the stability of the ferrite, the rate of breakdown can be slowed.

The ceramic particle-based system disclosed here has advantages over viral vectors, in that there is no risk of infection or of introduction of unwanted viral genes. It has advantages over the gold colloid system in that the particles are biodegradable. Further, since they may have a coat to which proteins may be covalently bound, these particles can be injected into the body via less drastic and more traditional pharmaceutical routes. The facility with which various proteins and enzymes can be bound to the particle surface makes them far more flexible and complex as delivery agents than simple lipid spheres.

The size of the particles as used in this invention is preferably 5 to 100 nm in diameter including all attached coatings and other surface molecules. Such particles can be filter-sterilised and subjected to affinity chromatography before DNA is loaded. This means that particle carriers can be sold independent of the DNA and can serve as a convenient synthetic vector for a wide variety of applications. It is also convenient to label the metal oxide core with radioactive emitters where this is useful to trace their distribution.

### Example

Use double distilled water (not de-ionised) to make up the reaction mixture. The following steps are conducted:
Add 1.5 ml of 33% NH₃ to 4.5 ml of hot dH₂O (to make up 7.5% NH₄OH) and leave standing in a capped universal tube in the water bath and bring to 60°C.

Dissolve 1.25 g Dextran (MW 10,000) in 2.0 ml of ddH₂O then dissolve 225 mg FeCl₃.6H₂O in the dextran solution. Alternatively, a trivalent lanthanide chloride may be substituted for 10 to 50% of the FeCl₃. When this is done, the subsequent post-reaction incubation is extended to two hours.

Dissolve 100 mg FeCl₂.4H₂O in the Fe₃/dextran solution then place the mixture in a 60°C water bath for two minutes before starting to gradually add 6 ml of hot 7.5% NH₃ solution (60°C). The product is left to stand in the 60°C water bath for fifteen minutes.

The reaction product (dextran-coated ferrites) is spun at 1,000 g for 10 minutes and any precipitate is discarded. This process is repeated to complete three spins and the supernatant then applied to PD-10 columns equilibrated with 0.1 M NaAcetate buffer, pH 6.8 with 5 mM EDTA.

The black eluted fraction is diluted 1:3 with EDTA/Acetate buffer then concentrated to one-tenth the initial volume with Amicon Centriprep-100 ultrafilters. The retentate is then diluted 1:10 with EDTA/Acetate buffer then concentrated to a volume of 1.5 ml with the C-100 ultrafilters.

Add 0.30 ml of 20 mM NaIO₄ to the dextran ferrite solution (approx. 1.5 ml) while stirring then gently tumble or shake for 60 minutes at room temperature in the dark.

At the end of the 60-minute periodate incubation, the reaction is terminated by applying the reaction mixture to the PD-10 columns equilibrated with 20 mM borate buffer (pH 8.5).

An active site blocking solution is prepared using 100 mM MnCl₂/CaCl₂ for WGA binding reactions. Alternatively, e.g. calf thymus DNA can be used where the protein active site to be protected is on a nucleic acid binding protein.

Dissolve 10 mg of the protein (e.g. DNAse free DNA pol 1, Klenow fragment, integrase, useful proteins for subsequent translation steps, nucleic acid packaging protein and anti-CD4, WGA, or other cell-targeting protein) in 500 µl of 20 mM Na borate buffer, pH 8.5 at room temperature. The protein solution can be diluted to 12 ml with borate buffer, then concentrated with Centriprep-10 concentrators to remove DTT, glycerol, NaN₃ and other undesirable storage additives.

Add 10 µl of the blocking solution to the protein/borate solution then mix 2.0 ml of oxidised magnetite dextran with 500 µl of the protein/borate solution. Pipette 20 µl of the blocking solution into the 2.5 ml protein-dextran-magnetite mixture and mix well, then incubate for 6 to 18 hours at room temperature in a gentle tumbling or shaking device.

After the incubation, add 100 µl of 0.5 M glycine to the reaction mixture and incubate an additional 2 hours. Then add 250 µl of 0.25 M NaBH₄ to the magnetite-dextran-protein solution and allow to stand for 60 minutes, shaking periodically to release H₂ gas. At the end of the incubation, pass the reaction mixture through PD-10 columns equilibrated with 20 mM HEPES buffer, pH 7.4. Dilute the eluant 1:5 with HEPES buffer then concentrate with Centriprep-100 ultrafilters.

An affinity purification step is optional and detail is given for use with a WGA(lectin) targeting protein. Apply final retentate to affinity columns (20 mM HEPES), wash with HEPES, then carry out specific elution with 1 M NAcGlu in HEPES buffer, pH 7.4. Pass the specific eluant through PD-10 columns equilibrated with HEPES to remove NAcGlu, Mn and Ca.

The desalted output is then diluted to a volume of 24 ml with HEPES buffer and concentrated with Centriprep-100 concentrators. The final retentate is sterilised by spinning at 500 h for one hour in 0.22 µm centrifugal microfilters.

The purified, sterilised synthetic vector particles can now be stored at 4°C for use within one to two weeks. They should not be frozen or lyophilised.

DNA adhesion with the DNA of interest can be done immediately prior to the transfection. The particle solutions are incubated with the DNA of interest with gentle tumbling or shaking for 6 to 24 hours.

Depending on the experimental or therapeutic protocol, the DNA-loaded vector solution may then be applied to cell cultures at a concentration of 1 mg/ml (approx. 5 mM Fe) of the synthetic vector (the final product of the preparation is 25 to 50 mg of synthetic vector). To assess efficiency, it may be compared to unadsorbed DNA solution. Alternatively, the DNA-loaded synthetic vector may be administered by IV or IM routes for in vivo use at 10 to 100 mM concentration. Non-precipitating magnetic-based separation techniques can be used to separate unbound DNA from particles. Where smaller DNA molecules are used, the separation can be done with Centriprep-100 concentrators.

## Claims

1. Ferrite particles obtainable by coprecipitation of ferrous and ferric salts from aqueous solution, which are free of hydrous oxides chelatable with EDTA, and which are coated with a biologically-tolerable polymer.

2. Ferrite particles according to claim 1, 5 to 100 nm in size.

3. Ferrite particles according to claim 1 or claim 2, wherein the biologically-tolerable polymer is dextran.

4. Ferrite particles according to any preceding claim, which carry DNA, RNA, plasmids, ribosomal particles or nucleic acid-binding proteins.

5. Ferrite particles according to any of claims 1 to 3, which carry a nerve adhesion molecule.

6. Ferrite particles according to any preceding claim, which exhibit superparamagnetism.

7. Ferrite particles according to any preceding claim, for therapeutic or diagnostic use.

8. A process for preparing ferrite particles according to any preceding claim, which comprises the coprecipitation, treatment with a chelating agent such as EDTA, and separation of the resultant chelate, and the biologically-tolerable polymer is present during coprecipitation or is subsequently coated on the particles.

## Patentansprüche

1. Ferrit-Teilchen erhältlich durch Copräzipitation von Eisen(II)- und Eisen(III)-Salzen aus wässriger Lösung, die frei von wasserhaltigen, mit EDTA chelatisierbaren Oxiden sind und mit einem biologisch verträglichen Polymer beschichtet sind.

2. Ferrit-Teilchen nach Anspruch 1 mit einer Größe von 5 bis 100 nrn.

3. Ferrit-Teilchen nach Anspruch 1 oder Anspruch 2, worin das biologisch verträgliche Polymer Dextran ist.

4. Ferrit-Teilchen nach irgendeinem vorangehenden Anspruch, welche DNA, RNA, Plasmide, ribosomale Teilchen oder Nucleinsäure-bindende Proteine tragen.

5. Ferrit-Teilchen nach irgendeinem der Ansprüche 1 bis 3, welche ein Nervenadhäsionsmolekül tragen.

6. Ferrit-Teilchen nach irgendeinem vorangehenden Anspruch, welche Superparamagnetismus zeigen.

7. Ferrit-Teilchen nach irgendeinem vorangehenden Anspruch für therapeutische oder diagnostische Zwecke.

8. Verfahren zur Herstellung von Ferrit-Teilchen nach irgendeinem vorangehenden Anspruch, welches umfasst die Copräzipitation, Behandlung mit einem Chelatbildner wie EDTA und Abtrennung des resultierenden Chelats, wobei das biologisch verträgliche Polymer während der Copräzipitation anwesend ist oder anschließend auf die Teilchen aufgebracht wird.

## Revendications

1. Particules de ferrite pouvant être obtenues par coprécipitation de sels ferreux et ferriques en solution aqueuse, qui sont dépourvues d'oxydes hydratés chélatables avec l'EDTA, et qui sont enrobées d'un polymère biologiquement compatible.

2. Particules de ferrite suivant la revendication 1, ayant des dimensions de 5 à 100 nm.

3. Particules de ferrite suivant la revendication 1 ou la revendication 2, dans lesquelles le polymère biologiquement compatible est le dextrane.

4. Particules de ferrite suivant l'une quelconque des revendications précédentes, qui portent un ADN, un ARN, des plasmides, des particules ribosomales ou des protéines de liaison aux acides nucléiques.

5. Particules de ferrite suivant l'une quelconque des revendications 1 à 3, qui portent une molécule d'adhésion neurale.

6. Particules de ferrite suivant l'une quelconque des revendications précédentes, qui sont douées de suprapara-magnétisme.

7. Particules de ferrite suivant l'une quelconque des revendications précédentes, destinées à une utilisation thérapeutique ou de diagnostic.

8. Procédé pour la préparation de particules de ferrite suivant l'une quelconque des revendications précédentes, qui comprend la coprécipitation, le traitement avec un agent chélatant tel que l'EDTA et la séparation du chélate résultant, le polymère biologiquement compatible étant présent au cours de la coprécipitation ou bien étant ensuite appliqué sous forme d'un revêtement sur les particules.
